# EUROPEAN PATENT APPLICATION

(11) **EP 4 467 530 A1**
(43) Date of publication of application: **27.11.2024**
(21) Application number: 23743160.6
(22) Date of filing: 12.01.2023
(51) Int. Cl.: C07C 29/141, C07B 61/00, C07C 29/76, C07C 29/80, C07C 35/37

(54) **PRODUCTION METHOD FOR POLYALCOHOL**

(30) Priority: 20.01.2022 JP 2022007310
(71) Applicant: Mitsubishi Chemical Corporation, Tokyo 100-8251 (JP)
(72) Inventor: TASHIMA, Naoto, Tokyo 100-8251 (JP); SUNAMI, Kazuaki, Tokyo 100-8251 (JP); HINOISHI, Hiroki, Tokyo 100-8251 (JP)
(74) Representative: Hoffmann Eitle
(86) International application number: PCT/JP2023/000562
(87) International publication number: WO 2023/140165

(57) **Abstract**

According to the present invention, a production method for a polyalcohol involves distillation refining a crude reaction liquid obtained by performing a reduction reaction on a starting material that is a polyaldehyde that has an alicyclic structure in the presence of a hydrogenating catalyst and hydrogen, the distillation refining being performed after the metal element content of the crude reaction liquid is at or below 30 mass ppm and preferably at or below 10 mass ppm.

## Description

### TECHNICAL FIELD

The present invention relates to a method for producing a polyhydric alcohol by subjecting a crude reaction solution obtained by performing a reduction reaction using a polyhydric aldehyde having an alicyclic structure as a starting material to distillation purification.

### BACKGROUND ART

There has been known a method for producing a polyhydric alcohol by performing a reduction reaction in the presence of a hydrogenation catalyst and hydrogen using a polyhydric aldehyde as a starting material.

Specifically, there is a method in which dicyclopentadiene used as an alkene is hydroformylated to obtain tricyclodecanedicarbaldehyde as a polyhydric aldehyde, and then the tricyclodecanedicarbaldehyde is hydrogenated to synthesize tricyclodecane dimethanol as a polyhydric alcohol (Patent Literature 1).

A distillation method is industrially used as a method for purifying a target high-boiling-point alcohol from a crude product containing a high-boiling-point alcohol as a polyhydric alcohol.

When the crude product contains a polyhydric aldehyde and a polyhydric alcohol, which have a boiling point close to that of the polyhydric alcohol (the high-boiling-point alcohol), the number of stages of a distillation column increases in order to separate and recover a high-purity polyhydric alcohol from the crude product, and it is necessary to increase a distillation column bottom temperature. In this case, the polyhydric alcohol is thermally decomposed, and a product recovery ratio is lowered, or a thermal decomposition product is mixed into the product.

In order to solve the problem, Patent Literature 1 discloses a technique of adding a sulfur compound to a crude product to prevent thermal decomposition of a high-boiling-point alcohol.

Patent Literature 2 discloses a technique in which, in order to separate an aldehyde condensate having a close boiling point and a high-boiling-point alcohol, an alkaline earth metal compound is added to a crude product to increase the weight of the aldehyde condensate to prevent distillation, thereby shortening a heating time and preventing thermal decomposition of the high-boiling-point alcohol.

Patent Literature 3 discloses a technique in which, in order to separate an aldehyde condensate having a close boiling point and a high-boiling-point alcohol, an acid is added to a crude product to increase the weight of the aldehyde condensate to prevent distillation, thereby shortening a heating time and preventing thermal decomposition of the high-boiling-point alcohol.

Patent Literatures 4 and 5 disclose a technique for preventing decomposition of a high-boiling-point alcohol by filtering out a hydrogenation catalyst in a crude reaction solution using a filter.

### CITATION LIST

### PATENT LITERATURE

Patent Literature 1: JPH11-60525A
Patent Literature 2: JP2002-47225A
Patent Literature 3: JP2003-192621A
Patent Literature 4: JP2004-196778A
Patent Literature 5: WO2011/064184

### SUMMARY OF INVENTION

### TECHNICAL PROBLEM

In the technique disclosed in Patent Literature 3, the added acid may corrode a reactor, a process pipe, etc.

In the techniques disclosed in Patent Literatures 1 to 3, there is a possibility that an additive such as a sulfur compound, an alkaline earth metal, or an acid may be mixed into a high-boiling-point alcohol product after distillation to contaminate the product.

Further, in order to use the additive, a facility for feeding the additive, a facility for removing the additive in some cases, etc. are required, which causes a problem of complicating a production process and increasing a production cost.

As in the techniques disclosed in Patent Literatures 4 and 5, simply filtering out the hydrogenation catalyst in the crude reaction solution using a filter cannot sufficiently prevent decomposition of the high-boiling-point alcohol during distillation.

An object of the present invention is to solve the problems.

An object of the present invention is to provide a method for producing a polyhydric alcohol capable of separating and recovering a high-purity polyhydric alcohol at a high yield by preventing thermal decomposition of the polyhydric alcohol when subjecting a crude reaction solution obtained by performing a reduction reaction using a polyhydric aldehyde having an alicyclic structure as a starting material to distillation purification.

### SOLUTION TO PROBLEM

The present inventor has found that the above problems can be solved by making a content of a metal element contained in the crude reaction solution before distillation equal to or less than a predetermined value.

The gist of the present invention is as follows.
[1] A method for producing a polyhydric alcohol, the method including:
   subjecting a crude reaction solution obtained by performing a reduction reaction using a polyhydric aldehyde having an alicyclic structure as a starting material to distillation purification, in which
   the distillation purification is performed after a content of a metal element in the crude reaction solution is 30 ppm by mass or less.
[2] The method for producing a polyhydric alcohol according to [1], in which
   the distillation purification is performed after the content of the metal element in the crude reaction solution is 10 ppm by mass or less.
[3] The method for producing a polyhydric alcohol according to [1] or [2], in which
   the reduction reaction is performed in the presence of a hydrogenation catalyst and hydrogen.
[4] The method for producing a polyhydric alcohol according to any one of [1] to [3], in which
   the polyhydric alcohol has a boiling point of 300°C or higher under a normal pressure.
[5] The method for producing a polyhydric alcohol according to [3] or [4], in which
   the metal element is a metal derived from the hydrogenation catalyst.
[6] The method for producing a polyhydric alcohol according to any one of [1] to [5], in which
   the metal element is a transition metal element belonging to a periodic table Group 4 and/or 5.
[7] The method for producing a polyhydric alcohol according to [6], in which
   the metal element is at least one selected from the group consisting of ruthenium, rhodium, palladium, and nickel.
[8] The method for producing a polyhydric alcohol according to any one of [1] to [7], in which
   the distillation purification is performed after a pH of the crude reaction solution is in a range of 6 to 8.
[9] The method for producing an alcohol according to any one of [1] to [8], in which
   the crude reaction solution is treated using an adsorbent to make the content of the metal element in the crude reaction solution 10 ppm by mass or less.
[10] The method for producing a polyhydric alcohol according to [9], in which
   the crude reaction solution is subjected to an activated carbon treatment to make the content of the metal element in the crude reaction solution 10 ppm by mass or less.
[11] The method for producing a polyhydric alcohol according to any one of [1] to [10], in which
   the distillation purification is performed after the content of the metal element in the crude reaction solution is 1 ppm by mass or less.
[12] The method for producing a polyhydric alcohol according to any one of [1] to [11], in which
   the polyhydric alcohol is tricyclo[5.2.1.0(2,6)]decane dimethanol.
[13] The method for producing a polyhydric alcohol according to any one of [1] to [12], the method including:
   a step of hydroformylating dicyclopentadiene to obtain tricyclodecanedicarbaldehyde as the polyhydric aldehyde; and
   a step of subjecting the tricyclodecanedicarbaldehyde to a reduction reaction in the presence of a hydrogenation catalyst and hydrogen to obtain a crude reaction solution containing tricyclo[5.2.1.0(2,6)]decane dimethanol.

### ADVANTAGEOUS EFFECTS OF INVENTION

According to the present invention, when subjecting a crude reaction solution obtained by performing a reduction reaction using a polyhydric aldehyde having an alicyclic structure as a starting material to distillation purification, thermal decomposition of a polyhydric alcohol can be prevented and a high-purity polyhydric alcohol can be separated and recovered at a high yield.

Furthermore, according to the present invention, distillation purification can be performed without using an additive such as a sulfur compound, an alkaline earth metal, or an acid as disclosed in Patent Literatures 1 to 3, so that mixing of the additive into a product polyhydric alcohol, corrosion of a production facility, and an increase in a production cost can be prevented.

### DESCRIPTION OF EMBODIMENTS

Hereinafter, the present invention will be described in detail.

A method for producing a polyhydric alcohol of the present invention is a method for producing a polyhydric alcohol by subjecting a crude reaction solution obtained by performing a reduction reaction using a polyhydric aldehyde having an alicyclic structure as a starting material to distillation purification. The distillation purification is performed after a content of a metal element in the crude reaction solution is 30 ppm by mass or less, and preferably 10 ppm by mass or less.

In the present invention, the polyhydric alcohol refers to an alcohol having two or more hydroxyl groups in the molecule.

In the present invention, the polyhydric aldehyde having an alicyclic structure, which is a starting material for polyhydric alcohol synthesis, is a polyhydric aldehyde having a structure having one or more cyclic hydrocarbon groups.

In the present invention, the polyhydric aldehyde refers to an aldehyde having two or more aldehyde groups in the molecule.

Specific examples of the polyhydric aldehyde include a polyhydric aldehyde having 6 to 20 carbon atoms, and more preferably 8 to 12 carbon atoms, which may have a substituent or a hetero atom and is alicyclic or has an aromatic skeleton. More specific examples of the polyhydric aldehyde include: alicyclic dialdehydes such as 1,3-cyclohexanedicarbaldehyde or 1,4-cyclohexanedicarbaldehyde, 3(4),8(9)-tricyclo[5.2.1.0]decanedicarbaldehyde, and 2(3),5(6)-bicyclo[2.2.1]heptane dicarbaldehyde; and aromatic dialdehydes such as terephthalaldehyde and isophthalaldehyde. The polyhydric aldehydes are not limited thereto.

Using the above-described polyhydric aldehyde as a raw material, corresponding cycloaliphatic diols such as 1,3-cyclohexanedimethanol or 1,4-cyclohexanedimethanol, 3(4),8(9)-tricyclo[5.2.1.0]decane dimethanol, and 2(3),5(6)-bicyclo[2.2.1]heptane dimethanol; and aromatic diols such as p-xylene diol and m-xylene diol can be produced. The aromatic polyols to be produced are not limited thereto.

When a distillation column bottom temperature during distillation is set to a high temperature, a polyhydric alcohol having a boiling point of 300°C or higher under a normal pressure is effective as the above-described polyhydric alcohol having an alicyclic structure.

In the method for producing a polyhydric alcohol of the present invention, the reduction reaction can be performed in the presence of a hydrogenation catalyst and hydrogen.

In the present invention, the hydrogenation catalyst used in the reduction reaction for polyhydric alcohol synthesis is preferably a transition metal catalyst belonging to a periodic table Group 4 and/or 5. For example, a Raney catalyst such as Raney nickel, Raney cobalt, and Raney copper, a supported catalyst in which a hydrogenated metal such as nickel, cobalt, platinum, palladium, rhodium, ruthenium, and copper is supported on a support such as silica earth, silica, alumina, silica alumina, clay, titania, zirconia, magnesia, calcia, lanthanum oxide, niobium oxide, and carbon, or a metal complex catalyst containing a metal such as nickel, cobalt, platinum, palladium, rhodium, ruthenium, and copper and an organic or inorganic ligand can be used.

Among them, a hydrogenation catalyst containing ruthenium, rhodium, palladium, and nickel is particularly preferred from the viewpoint of hydrogenation catalyst activity, a catalyst cost, and catalyst separability, and a ruthenium catalyst is particularly preferred.

One type of the hydrogenation catalyst may be used alone, or two or more types thereof may be used in any combination.

Conditions such as a temperature, a hydrogen pressure, a solvent, a reaction system, and a reaction device in the reduction reaction for synthesizing the polyhydric alcohol are not particularly limited, and known methods are appropriately combined. As specific conditions, conditions described below for a hydrogenation reduction reaction of tricyclodecanedicarbaldehyde can be adopted.

The hydrogenation catalyst contained in the reaction product solution containing the polyhydric alcohol obtained by such a hydrogenation reduction reaction is removed by general methods such as filtration, adsorption, and extraction.

In the present invention, the distillation purification is performed using a solution after removing the hydrogenation catalyst as the crude reaction solution. Prior to the distillation purification, a metal element removal treatment is performed such that the content of the metal element in the crude reaction solution is 30 ppm by mass or less, and preferably 10 ppm by mass or less.

The crude reaction solution obtained by the hydrogenation reduction reaction according to the present invention contains a metal element derived from the hydrogenation catalyst. The content of the metal element in the crude reaction solution varies depending on the amount of the hydrogenation catalyst used in the hydrogenation reduction reaction, and is usually about 10 mass ppm to 100 mass ppm.

The present inventor has found that the metal element derived from the hydrogenation catalyst causes decomposition of the polyhydric alcohol due to heating in the distillation purification step, thereby causing a decrease in yield and purity of the polyhydric alcohol.

In the present invention, prior to the distillation purification, the metal element in the crude reaction solution is removed to prevent the thermal decomposition of the polyhydric alcohol caused by the metal element in the distillation purification step.

A method for removing the metal element in the crude reaction solution to reduce the content thereof is not particularly limited. For example, a method of treating using a known adsorbent is exemplified. Specific examples of the treatment using an adsorbent include an activated carbon treatment, and a treatment using a cation exchange resin, silica gel adsorption, etc. The activated carbon treatment is preferred due to removal efficiency and the possibility of reusing the adsorbent.

The method using the activated carbon treatment may be a batch treatment in which activated carbon is added to the crude reaction solution and stirred, and then the activated carbon is subjected to solid-liquid separation by filtration, etc., or may be a continuous treatment in which the crude reaction solution is passed through an activated carbon-packed column.

In the case of the batch treatment, the amount of activated carbon to be added to the crude reaction solution is appropriately determined depending on the metal element adsorption ability of the activated carbon, the content of the metal element in the crude reaction solution, etc. Under general conditions, it is preferable to add the activated carbon to the crude reaction solution to have a concentration of about 0.01 mass% to 10 mass%, followed by stirring.

In the case of the continuous treatment, a treatment flow rate is not particularly limited, and may be 1 to 10 at a space velocity (LHSV).

Such an activated carbon treatment may be performed a plurality of times. That is, the activated carbon treatment may be performed again on an activated carbon treatment solution obtained by performing the activated carbon treatment on the crude reaction solution. In this case, the type, the amount, the treatment conditions, etc. of the activated carbon used in a first activated carbon treatment and a second activated carbon treatment may be changed.

In the present invention, the distillation purification is performed after the content of the metal element in the crude reaction solution is made 30 ppm by mass or less, and preferably 10 ppm by mass or less by subjecting the crude reaction solution to the activated carbon treatment, etc.

The lower the content of the metal element in the crude reaction solution to be subjected to the distillation purification, the more preferable from the viewpoint of preventing the thermal decomposition of the polyhydric alcohol. The content of the metal element in the crude reaction solution to be subjected to the distillation purification is particularly preferably 5 ppm by mass or less, and particularly preferably 1 ppm by mass or less.

The pH of the crude reaction solution to be subjected to the distillation purification is preferably in a range of 6 to 8. It is preferable that the lower limit of the pH is 6 or more, since a byproduct of a compound having a boiling point lower than that of a target product, which is thought to be caused by dehydration of the product alcohol, and a byproduct of a compound having a boiling point higher than that of the target product, which is thought to be caused by dimerization such as etherification, can be prevented. On the other hand, it is preferable that the upper limit of the pH is 8 or less, since a distillation purification facility is less likely to be subjected to alkali corrosion.

Usually, the pH of the reaction product solution obtained by the hydrogenation reduction reaction of polyhydric aldehyde is 6 to 8, and the pH hardly changes even when the reaction product solution is subjected to the hydrogenation catalyst removal treatment and the metal element removal treatment. However, the pH may deviate from the range of 6 to 8 due to an acid and an alkali component eluted from the hydrogenation catalyst. In this case, the pH is preferably adjusted to pH 6 to 8 by appropriately adding a pH adjusting agent such as an acid or an alkali.

The crude reaction solution having a content of the metal element of 30 ppm by mass or less and preferably 10 ppm by mass or less is then subjected to the distillation purification.

The distillation purification conditions are not particularly limited, and usually, a person skilled in the art can perform the distillation purification by appropriately optimizing the distillation purification conditions according to the purpose using a distillation column having a theoretical number of stages of 1 to 30, a distillation column bottom temperature of 150°C to 250°C, a pressure of 0.1 kPa to 100 kPa, and a reflux ratio of 1 to 30.

By performing the distillation purification by the method described above, usually, a polyhydric alcohol having a purity of 98% or more can be obtained with a high yield.

Such a method for producing a polyhydric alcohol of the present invention is particularly effective for producing tricyclo[5.2.1.0(2,6)]decane dimethanol by hydrogenation reduction of tricyclodecanedicarbaldehyde, since the tricyclo[5.2.1.0(2,6)]decane dimethanol is a high-boiling-point compound that requires high temperature conditions during distillation and is not solid at room temperature, making it difficult to perform another purification such as crystallization.

A method for producing tricyclo[5.2.1.0(2,6)]decane dimethanol employing the present invention will be described below.

### [Method for Producing Tricyclo[5.2.1.0(2,6)]decane Dimethanol]

The method for producing tricyclo[5.2.1.0(2,6)]decane dimethanol according to the present invention includes: a step of hydroformylating dicyclopentadiene serving as a polyhydric aldehyde as a starting material to obtain tricyclodecanedicarbaldehyde; and a step of subjecting the tricyclodecanedicarbaldehyde to a reduction reaction in the presence of a hydrogenation catalyst and hydrogen to obtain a crude reaction solution containing tricyclo[5.2.1.0(2,6)]decane dimethanol. As described above, after the content of the metal element in the crude reaction solution is 30 ppm by mass or less, and preferably 10 ppm by mass or less, the distillation purification is performed as described above.

### <Hydroformylation Reaction of Dicyclopentadiene>

The method for hydroformylation of dicyclopentadiene is not particularly limited, and the hydroformylation of dicyclopentadiene can be performed according to an ordinary method.

For example, according to a method described in JP2001-10999A, in the presence of a catalyst containing a rhodium compound and an organophosphorus compound in a hydroformylation reaction solvent containing a hydrocarbon compound, dicyclopentadiene is hydroformylated as in the following reaction formula (I) using hydrogen and carbon monoxide to produce tricyclodecanedicarbaldehyde.

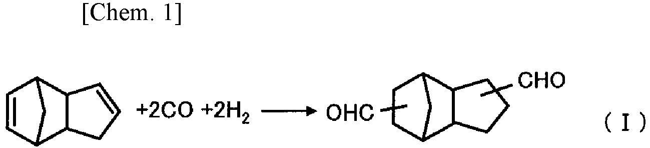

The rhodium compound to be used in the hydroformylation step does not depend on a form of a precursor as long as it forms a complex with the organophosphorus compound and exhibits hydroformylation activity in the presence of hydrogen and carbon monoxide.

A catalyst precursor substance such as Rh(acac)(CO)₂, Rh₂O₃, Rh₄(CO)₁₂, Rh₆(CO)₁₆, and Rh(NO₃)₃ may be introduced into a reaction mixture together with the organophosphorus compound to form a rhodium metal hydride carbonyl phosphorus complex having catalytic activity in the reactor, or a rhodium metal hydride carbonyl phosphorus complex catalyst may be prepared in advance and introduced into the reactor.

In a preferred specific example of the present invention, Rh(acac)(CO)₂ is used as a rhodium precursor substance to react with an organophosphorus compound in the presence of a solvent, followed by introducing into a reactor together with an excess free organophosphorus compound to form a rhodium-organophosphorus compound complex catalyst having catalytic activity.

Examples of the organophosphorus compound that forms a catalyst for the hydroformylation reaction with the rhodium compound include phosphites and phosphines.

Among them, a compound represented by a general formula P(-OR¹)(-OR²)(-OR³) (in the formula, R¹, R², and R³ each represent an aryl group or an alkyl group which may be substituted) is preferred as the phosphite since it is effective for the hydroformylation reaction of dicyclopentadiene. Specific examples of R¹, R², and R³ include: aryl groups such as a phenyl group and a naphthyl group which may be substituted with a methyl group, an ethyl group, an isopropyl group, an n-butyl group, a t-butyl group, and a methoxy group; aliphatic alkyl groups such as a methyl group, an ethyl group, an isopropyl group, an n-butyl group, and a t-butyl group; and alicyclic alkyl groups such as a cyclopentyl group and a cyclohexyl group which may be substituted with a lower alkyl group such as a methyl group, an ethyl group, an isopropyl group, an n-butyl group, and a t-butyl group.

Specific examples of the suitable phosphite include tris(2-t-butylphenyl) phosphite, tris(3-methyl-6-t-butylphenyl) phosphite, tris(3-methoxy-6-t-butylphenyl) phosphite, tris(2,4-di-t-butylphenyl) phosphite, and di(2-t-butylphenyl)(t-butyl) phosphite. The phosphites are not limited thereto. One type of the phosphite may be used alone, or two or more types thereof may be used in any combination.

As the phosphines, sterically hindered alkyl phosphines are particularly effective in the hydroformylation reaction of dicyclopentadiene. Typical examples thereof include tricyclopropylphosphine, tricyclobutylphosphine, tricyclopentylphosphine, tricyclohexylphosphine, tricycloheptylphosphine, and tricyclooctylphosphine. The phosphines are not limited thereto. One type of the phosphine may be used alone, or two or more types thereof may be used in any combination.

For the amount of the organophosphorus compound to be used, when the organophosphorus compound is present in the hydroformylation reaction solution in an amount of 1 time to 400 times, and preferably 3 times to 200 times the amount of a rhodium metal by mole, tricyclodecanedicarbaldehyde can be obtained at a sufficient hydroformylation reaction rate.

The hydroformylation reaction of dicyclopentadiene can be performed without using a solvent, and can be more suitably performed by using an inactive organic solvent for the reaction.

As described later, after the completion of the hydroformylation reaction, the reaction product solution containing tricyclodecanedicarbaldehyde is brought into contact with alcohol, and the tricyclodecanedicarbaldehyde is extracted into an extraction solvent layer containing alcohol while the catalyst component is left in a dihydroformylation reaction solvent layer, thereby performing layer separation. Therefore, the hydroformylation reaction solvent is preferably a solvent capable of performing layer separation with alcohol. Examples of such a solvent include an aromatic hydrocarbon compound, an aliphatic hydrocarbon compound, and an alicyclic hydrocarbon compound.

As the aromatic hydrocarbon compound, benzene, methylbenzenes such as toluene, xylene, mesitylene, and pseudocumene, ethylbenzenes such as ethylbenzene, diethylbenzene, and triethylbenzene, propylbenzenes such as isopropylbenzene, 1,3-diisopropylbenzene, and 1,4-diisopropylbenzene, and various other alkylbenzenes can be suitably used.

Examples of the aliphatic hydrocarbon compound include pentane, hexane, heptane, octane, isooctane, dodecane, and decane. The aliphatic hydrocarbon compound is not limited thereto as long as it is a liquid at a standard temperature and pressure.

As the alicyclic hydrocarbon compound, cyclohexane, cyclooctane, cyclododecane, decalin, and methylcyclohexane can be suitably used.

An amount of the rhodium catalyst to be used is usually 10 mass ppm to 5000 mass ppm, and preferably 50 mass ppm to 2000 mass ppm, in terms of rhodium metal, with respect to dicyclopentadiene as a raw material. When rhodium is used at 50 ppm or more, it is necessary to recover the catalyst.

For a temperature and pressure for the hydroformylation reaction of dicyclopentadiene, the reaction temperature is usually 40°C to 160°C, and preferably 80°C to 140°C, and the reaction pressure is usually 1 MPa to 15 MPa. When the temperature is lower than 40°C, the hydroformylation reaction is slow, and when the temperature is higher than 160°C, a side reaction from dicyclopentadiene or a hydroformylation reaction product in the reaction solution proceeds, and the yield of aldehyde decreases. When the pressure is lower than 1 MPa, the hydroformylation reaction is slow, and when the pressure is higher than 15 MPa, a high-pressure reaction device is used, which increases an equipment cost.

A molar ratio of hydrogen to carbon monoxide in a hydrogen/carbon monoxide mixed gas used for the reaction can be selected from a range of 0.2 to 5.0 for a composition of the introduced gas (hydrogen/carbon monoxide). When the hydrogen/carbon monoxide mixed gas composition is out of this range, the reaction activity of the hydroformylation reaction or the aldehyde selectivity decreases.

As the hydroformylation reaction system, a continuous feed system is adopted in which dicyclopentadiene as a raw material is fed alone or a mixed solution of dicyclopentadiene and a solvent is fed to a reactor in which a rhodium-organophosphorus complex catalyst, a solvent, and a mixed gas of hydrogen and carbon monoxide are present. When the method is used, generation of cyclopentadiene that inhibits the hydroformylation reaction due to thermal decomposition of dicyclopentadiene in the reactor can be reduced, and a good reaction rate and yield can be maintained. In order to maintain the fluidity of dicyclopentadiene, it is preferable to dilute dicyclopentadiene with the solvent described above and feed dicyclopentadiene to the reactor at a temperature at which the dicyclopentadiene is not depolymerized to produce cyclopentadiene.

### <Extraction of Tricyclodecanedicarbaldehyde>

After the completion of the hydroformylation reaction, the reaction product solution is directly used, or is diluted with the hydrocarbon compound used in the reaction as the hydroformylation reaction solvent or another hydrocarbon compound, and then brought into contact with alcohol to extract tricyclodecanedicarbaldehyde as a product to the alcohol while leaving the catalyst component in the hydroformylation reaction solvent layer, thereby performing layer separation.

Examples of the alcohol include a primary alcohol having 1 to 3 carbon atoms and a polyhydric alcohol having 2 to 6 carbon atoms.

Examples of the primary alcohol having 1 to 3 carbon atoms include methanol, ethanol, and propanol.

Examples of the polyhydric alcohol having 2 to 6 carbon atoms include ethylene glycol, 1,3-propanediol, 1,2-propanediol, 1,4-butanediol, 1,2-butanediol, 1,3-butanediol, 2,3-butanediol, isomers of pentanediol, neopentyl glycol, hexanediol, glycerin, pentaerythritol, and trimethylolpropane.

Among them, methanol, ethylene glycol, propane diol, and butanediol are suitably used since they have relatively low boiling points, are inexpensive, and are easily handled as a liquid.

One type of the extraction solvent may be used alone, or two or more types thereof may be used in any combination.

The extraction may be performed with water coexisting with alcohol. The aldehyde and the catalyst component are more easily distributed to each layer by the addition of water.

The reaction solvent to be used in the hydroformylation reaction and the extraction solvent are preferably different in density in order to implement effective layer separation. One suitable example of a combination of the hydroformylation reaction solvent containing tricyclodecanedicarbaldehyde and the extraction solvent is a combination of methylcyclohexane and ethylene glycol, or methylcyclohexane and methanol, and further water.

The distribution of tricyclodecanedicarbaldehyde between the hydroformylation reaction solvent and the extraction solvent is equilibrium. On the other hand, rhodium and an organophosphorus compound, which are catalyst components, exist substantially only in the hydroformylation reaction solvent, and exist in the extraction solvent in an amount below the analysis limit.

A volume ratio of the extraction solvent to be used and the reaction product solution is determined by solubility of tricyclodecanedicarbaldehyde to the extraction solvent and the amount of tricyclodecanedicarbaldehyde to be extracted. For example, in the case where tricyclodecanedicarbaldehyde to be separated exhibits high solubility in the extraction solvent and is present in the reaction product solution at a low concentration, tricyclodecanedicarbaldehyde can be practically extracted by using the extraction solvent at a low volume ratio (extraction solvent/reaction product solution).

The higher the concentration of the product is, the higher the volume ratio (extraction solvent/reaction product solution) for extracting tricyclodecanedicarbaldehyde from the reaction product solution is. When tricyclodecanedicarbaldehyde exhibits a relatively low solubility in the extraction solution, the volume ratio may vary in a range of 10:1 to 1:10.

In order to increase the extraction amount of tricyclodecanedicarbaldehyde with a small amount of the extraction solvent to be used, it is effective to separate the extraction solvent and perform the extraction operation several times.

In this case, in the final extraction operation, the hydroformylation reaction solvent such as methylcyclohexane may be added in an amount of about 5 mass% to 20 mass% to the reaction product solution. A removal rate of the catalyst can be improved by adding the hydroformylation reaction solvent.

A temperature at which the extraction operation is performed is not particularly limited, and it is practical to perform the extraction operation at a temperature equal to or lower than a hydroformylation reaction temperature. After the reaction in the hydroformylation reactor, the extraction solvent may be added to perform the extraction operation. The hydroformylation reaction product solution may be taken out from the hydroformylation reactor, and the extraction operation may be performed in an extraction tank. The extraction operation can be performed by adding the extraction solvent directly to the hydroformylation reactor, and a next hydroformylation reaction can be performed while retaining the catalyst component in the hydroformylation reactor. When the hydroformylation reaction product solution is taken out and the operation is performed in the extraction tank, the hydrocarbon solvent layer containing the catalyst is returned to the hydroformylation reactor and used again in the reaction. The present process can be performed as a batch process or a continuous process.

In the extraction operation as described above, a tricyclodecanedicarbaldehyde-containing solution containing 10 mass% to 90 mass% of tricyclodecanedicarbaldehyde and 10 mass% to 90 mass% of the extraction solvent can be obtained. When the reaction solvent is added, a tricyclodecanedicarbaldehyde-containing solution containing 5 mass% to 90 mass% of tricyclodecanedicarbaldehyde, 5 mass% to 90 mass% of the extraction solvent, and 5 mass% to 90 mass% of the reaction solvent can be obtained.

The alcohol as the extraction solvent reacts with part of tricyclodecanedicarbaldehyde, which is a hydroformylated product, to produce an acetal compound by acetalizing the tricyclodecanedicarbaldehyde.

A content of the acetal compound in the tricyclodecanedicarbaldehyde is usually about 0.1 mass% to 50 mass%, and more preferably about 1 mass% to 25 mass%.

### <Hydrogenation Reduction Reaction>

An extraction solution containing the tricyclodecanedicarbaldehyde obtained by the above extraction operation (a tricyclodecanedicarbaldehyde-containing solution) is then subjected to hydrogenation reduction in the presence of the above hydrogenation catalyst, preferably a ruthenium (Ru) catalyst, to produce tricyclodecane dimethanol as shown in the following reaction formula (II).

The hydrogenation reduction reaction is performed in the presence of water and a Ru catalyst, thereby rapidly converting the acetal compound into tricyclodecanedicarbaldehyde during the hydrogenation reaction of tricyclodecanedicarbaldehyde. Tricyclodecane dimethanol can be produced at a high yield by hydrogenating tricyclodecanedicarbaldehyde converted from an acetal compound, which is preferable.

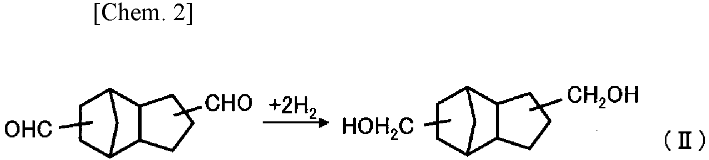

The amount of water present in the hydrogenation reduction reaction is preferably equal to or greater than the amount of the acetal compound in the hydrogenation reduction reaction solution, and is preferably an amount that does not cause phase separation of the reaction solution. The water present in the hydrogenation reduction reaction is preferably 2 mass% or more, preferably 2 mass% to 30 mass%, more preferably 5 mass% to 25 mass%, and particularly preferably 10 mass% to 20 mass%, in terms of water content, with respect to the entire reaction solution. When the water content is within the above range, the above-described effect can be effectively obtained by allowing water to be present in the hydrogenation reaction system without causing layer separation between water and the reaction solvent. This water may be added in the extraction step of separating the catalyst component and the polyhydric aldehyde from the hydroformylation reaction product solution, or may be added to the reaction system immediately before the hydrogenation reduction reaction.

As the reaction form, a method in which a slurry of a catalyst is charged into a stirring reactor, a reaction is performed in a batch manner, and after the reaction, the catalyst is precipitated and filtered to be separated from a product solution, and a perfusion type reaction in which a molded catalyst is charged into a tube type reactor and a product solution and a hydrogen gas flow on the catalyst are appropriately adopted. The amount of the catalyst to be used is not particularly limited as long as tricyclodecane dimethanol can be produced with industrially advantageous productivity.

For a reaction temperature and pressure for the hydrogenation reduction reaction, the temperature is usually 40°C to 200°C, and preferably 70°C to 150°C, and the reaction pressure is usually 15 MPa or less. When the temperature is lower than 40°C, the hydrogenation reduction reaction is slow, and when the temperature is higher than 200°C, a side reaction from the target product tricyclodecane dimethanol proceeds, resulting in a decrease in yield of tricyclodecane dimethanol. When the pressure is higher than 15 MPa, a high-pressure reaction device is used, which increases the equipment cost.

The crude reaction solution containing tricyclodecane dimethanol that is obtained in this way is subjected to a metal element content reduction treatment such as an activated carbon treatment after removing the hydrogenation catalyst as described above, and then subjected to the distillation purification.

### Examples

Hereinafter, the present invention will be described in more detail with reference to Examples. The present invention is not limited to the following Examples as long as the scope of the present invention is not exceeded.

Compounds used in Examples and Comparative Examples are as follows.
- Acetylacetonate dicarbonyl rhodium (trade name: Rh(acac)(CO)₂, manufactured by N.E. CHEMCAT CORPORATION)
- Ruthenium-supported carbon (dry base Ru content 5%, water content 56%) (trade name: Ru/C, manufactured by N.E. CHEMCAT CORPORATION)
- Tris(2,4-di-tert-butylphenyl) phosphite (trade name: DBPO, manufactured by Tokyo Chemical Industry Co., Ltd.)
- Methylcyclohexane (trade name: Methylcyclohexane, manufactured by FUJIFILM Wako Pure Chemical Corporation)
- Dicyclopentadiene (manufactured by FUJIFILM Wako Pure Chemical Corporation)
- Powder activated carbon A (trade name: SHIRASAGI ANO-2, manufactured by Osaka Gas Chemicals Co., Ltd.)
- Powder activated carbon B (trade name: characteristic SHIRASAGI, manufactured by Osaka Gas Chemicals Co., Ltd.)

### [Reference Example 1]

### <Hydroformylation Reaction>

An autoclave reactor having an internal volume of 20 L was charged with, under a nitrogen atmosphere, 2.51 g (9.73 mmol) of Rh(acac)(CO)₂ and 188.6 g (0.291 mol) of tris(2,4-di-t-butylphenyl) phosphite as raw material compounds of a hydroformylation reaction catalyst, and 3.9 kg of methylcyclohexane as an organic solvent. Thereafter, a temperature of a reaction solution in the reactor was increased to 70°C while stirring at 120 rpm. Next, a mixed gas of hydrogen and carbon monoxide (hydrogen:carbon monoxide = 1:1 (molar ratio)) was rapidly injected from a gas introduction valve such that the pressure in the reactor was 5 MPaG, and the temperature of the reaction solution was increased to 100°C while maintaining the pressure. Thereafter, 4.9 kg of dicyclopentadiene as a raw material compound was further fed over 5 hours, and the reaction was continued for further 3 hours. During the reaction, the amount of the mixed gas consumed in the reaction was continuously introduced into the reactor while maintaining the pressure in the reactor at 5 MPaG.

After completion of the reaction, the reaction solution in the reactor was cooled to room temperature, and the residual gas in the reactor was released to obtain 12.5 kg of a hydroformylation reaction product solution. The amount of dicyclopentadiene as the raw material compound contained in the reaction solution before the reaction and the amount of tricyclodecanedicarbaldehyde produced as a product in the reaction product solution after the reaction were analyzed by gas chromatography, the yield of tricyclodecanedicarbaldehyde was determined, and as a result, the yield was 99%.

### <Extraction Operation>

To 12.5 kg of the obtained hydroformylation reaction product solution, 3.77 kg of methanol and 2 kg of water were added, followed by stirring for 30 minutes under a nitrogen atmosphere. Thereafter, the mixture was allowed to stand for 30 minutes to separate into two phases, and an extraction operation was performed. To the obtained lower phase (a1), 0.4 kg of methylcyclohexane was added, followed by stirring for 30 minutes. Thereafter, the mixture was allowed to stand for 30 minutes to separate into two phases, and an extraction operation was performed to obtain 13.8 kg of the lower phase (a2).

A composition of the obtained lower phase (a2) was analyzed by gas chromatography and found to be 47 mass% of tricyclodecanedicarbaldehyde, 27 mass% of methanol, 14 mass% of water, 7 mass% of methylcyclohexane, and 5 mass% of other components.

### <Hydrogenation Reduction Reaction>

An autoclave reactor having an internal volume of 20 L was charged with 13.8 kg of the lower phase (a2) obtained by the above-described extraction operation and 0.14 kg of ruthenium-supported carbon, and then a temperature of the reaction solution in the reactor was increased to 160°C while stirring at 120 rpm. Next, a hydrogen gas was injected from a gas introduction valve such that the pressure in the reactor was 5 MPaG, and the reaction was performed for 8 hours while maintaining the pressure and the temperature of the reaction solution. During the reaction, the amount of the hydrogen gas consumed in the reaction was continuously introduced into the reactor while maintaining the pressure in the reactor at 5 MPaG.

After the completion of the reaction, the reaction solution in the reactor was cooled to room temperature, the residual gas in the reactor was released, and the ruthenium-supported carbon was separated by filtration using a 5-µm filter to obtain 14.5 kg of a reaction product solution.

The amount of tricyclodecanedicarbaldehyde as the raw material compound contained in the reaction solution before the reaction and the amount of tricyclo[5.2.1.0(2,6)]decane dimethanol (hereinafter referred to as "TCDDM") produced as a product in the reaction product solution after the reaction were analyzed by gas chromatography, and as a result, the yield of TCDDM was 94%.

The Ru concentration in the reaction product solution analyzed by fluorescent X-ray analysis was 36 mass ppm, and the pH measured by the pH test paper was 7. This reaction product solution is referred to as a "crude reaction product solution".

### [Example 1]

Powder activated carbon A was added to 100 g of the crude reaction product solution obtained in Reference Example 1 so as to have a content concentration of 1 mass%, followed by stirring at room temperature for 3 hours. Next, the reaction product solution after stirring was filtered to remove the powder activated carbon A, thereby obtaining 95 g of an activated carbon treatment solution.

As a result of analysis by gas chromatography, a composition of the activated carbon treatment solution was 52 mass% of TCDDM, 25 mass% of methanol, 14 mass% of water, 4 mass% of methylcyclohexane, and 5 mass% of other components.

The Ru concentration of the activated carbon treatment solution analyzed by fluorescent X-ray analysis was 3.5 mass ppm, and the pH measured by the pH test paper was 7.

As a model experiment of distillation, the solvent in the obtained activated carbon treatment solution was distilled off under reduced pressure, followed by heating at 230°C, which is the distillation temperature when distilling TCDDM, for 5 hours and then cooling to room temperature. The amounts of TCDDM contained in the activated carbon treatment solution after solvent distillation under reduced pressure before heating and after heating were analyzed by gas chromatography, and as a result, the residual ratio of TCDDM was 94 mass%.

### [Example 2]

Powder activated carbon B was added to 100 g of the crude reaction product solution obtained in Reference Example 1 so as to have a content concentration of 1 mass%, followed by stirring at room temperature for 3 hours. Next, the reaction product solution after stirring was filtered to remove the powder activated carbon B, thereby obtaining 95 g of an activated carbon treatment solution.

A composition of the activated carbon treatment solution analyzed in the same manner as in Example 1 was 52 mass% of TCDDM, 25 mass% of methanol, 14 mass% of water, 4 mass% of methylcyclohexane, and 5 mass% of other components. The activated carbon treatment solution had a Ru concentration of 0.7 mass ppm and a pH of 7.

As a model experiment of distillation, in the same manner as in Example 1, the solvent in the obtained activated carbon treatment solution was distilled off under reduced pressure, followed by heating at 230°C, which is the distillation temperature when distilling TCDDM, for 5 hours, and then cooling to room temperature, and the residual ratio of TCDDM after heating was analyzed, and as a result, the residual ratio of TCDDM was 97 mass%.

### [Comparative Example 1]

As a model experiment of distillation, in the same manner as in Example 1 without subjecting 100 g of the crude reaction product solution having a Ru concentration of 36 ppm and obtained in Reference Example 1 to the activated carbon treatment, the solvent was distilled off under reduced pressure, followed by heating, and the residual ratio of TCDDM after heating was analyzed, and as a result, the residual ratio of TCDDM was 56 mass%.

The evaluation results in Examples 1 and 2 and Comparative Example 1 were summarized in Table 1.

**[Table 1]**

| | Activated carbon treatment | Ru concentration (mass ppm) | TCDDM residual ratio (mass%) |
|---|---|---|---|
| Example 1 | Powder activated carbon A | 3.5 | 94 |
| Example 2 | Powder activated carbon B | 0.7 | 97 |
| Comparative Example 1 | No | 36 | 56 |

In Example 1 and Example 2, since the content of the metal element (Ru) contained in the activated carbon treatment solution before solvent distillation under reduced pressure was 30 mass ppm or less, the residual ratio of TCDDM after heating at 230°C, which is the distillation temperature when distilling TCDDM, for 5 hours was high.

On the other hand, in Comparative Example 1, since the content of the metal element (Ru) contained in the crude reaction product solution was more than 30 mass ppm, the residual ratio of TCDDM after heating at 230°C, which is the distillation temperature when distilling TCDDM, for 5 hours was low.

It was confirmed from the results in Example 1, Example 2, and Comparative Example 1 that the residual ratio of TCDDM tends to increase as the content of the metal element (Ru) decreases.

### [Example 3]

To 14.5 kg of the crude reaction product solution obtained in Reference Example 1, 0.15 kg of the powder activated carbon B was added and stirred for 3.5 hours, and then the stirring was stopped, followed by filtering to obtain 13 kg of a first activated carbon treatment solution. After 0.13 kg of the powder activated carbon B was added again to the first activated carbon treatment solution and stirred for 3 hours, followed by filtering to obtain 12 kg of a second activated carbon treatment solution. The Ru concentration in the second activated carbon treatment solution that is analyzed in the same manner as in Example 1 was 0.4 mass ppm, and the pH was 7.

As a result of gas chromatography analysis, a composition of the second activated carbon treatment solution was 45 mass% of tricyclodecane dimethanol, 29 mass% of methanol, 14 mass% of water, 7 mass% of methyl cyclohexane, and 5 mass% of other components.

A batch distillation device having a pot volume of 35 L was charged with 12 kg of the second activated carbon treatment solution, and the solvent was distilled off, followed by distillation at 0.3 kPa and 210°C to obtain 9.5 kg of TCDDM with a purity of 99.4 mass%.

In a series of distillation, the amounts of TCDDM contained in the distillation raw material solution (the second activated carbon treatment solution) before heating, the distillate solution after heating, and the column bottom solution were analyzed by gas chromatography, and as a result, the residual ratio of TCDDM was 99 mass%.

From the above results, according to the production method of the present invention, it is expected that by making the content of the metal element (Ru) in the crude reaction solution subjected to the distillation 30 ppm by mass or less, and preferably 10 ppm by mass or less when distilling TCDDM, the loss due to the decomposition of TCDDM can be reduced, and high-purity TCDDM can be produced at a high yield.

Although the present invention has been explained in detail using specific embodiments, it is obvious to those skilled in the art that various modifications can be made without departing from the spirit and the scope of the present invention.

The present application is based on a Japanese patent application filed on January 20, 2022 (patent application No. 2022-007310), which is hereby incorporated by reference in its entirety.

## Claims

1. A method for producing a polyhydric alcohol, the method comprising:
subjecting a crude reaction solution obtained by performing a reduction reaction using a polyhydric aldehyde having an alicyclic structure as a starting material to distillation purification, wherein
the distillation purification is performed after a content of a metal element in the crude reaction solution is 30 ppm by mass or less.

2. The method for producing a polyhydric alcohol according to claim 1, wherein
the distillation purification is performed after the content of the metal element in the crude reaction solution is 10 ppm by mass or less.

3. The method for producing a polyhydric alcohol according to claim 1 or 2, wherein
the reduction reaction is performed in the presence of a hydrogenation catalyst and hydrogen.

4. The method for producing a polyhydric alcohol according to any one of claims 1 to 3, wherein
the polyhydric alcohol has a boiling point of 300°C or higher under a normal pressure.

5. The method for producing a polyhydric alcohol according to claim 3 or 4, wherein
the metal element is a metal derived from the hydrogenation catalyst.

6. The method for producing a polyhydric alcohol according to any one of claims 1 to 5, wherein
the metal element is a transition metal element belonging to a periodic table Group 4 and/or 5.

7. The method for producing a polyhydric alcohol according to claim 6, wherein
the metal element is at least one selected from the group consisting of ruthenium, rhodium, palladium, and nickel.

8. The method for producing a polyhydric alcohol according to any one of claims 1 to 7, wherein
the distillation purification is performed after a pH of the crude reaction solution is in a range of 6 to 8.

9. The method for producing an alcohol according to any one of claims 1 to 8, wherein
the crude reaction solution is treated using an adsorbent to make the content of the metal element in the crude reaction solution 10 ppm by mass or less.

10. The method for producing a polyhydric alcohol according to claim 9, wherein
the crude reaction solution is subjected to an activated carbon treatment to make the content of the metal element in the crude reaction solution 10 ppm by mass or less.

11. The method for producing a polyhydric alcohol according to any one of claims 1 to 10, wherein
the distillation purification is performed after the content of the metal element in the crude reaction solution is 1 ppm by mass or less.

12. The method for producing a polyhydric alcohol according to any one of claims 1 to 11, wherein
the polyhydric alcohol is tricyclo[5.2.1.0(2,6)]decane dimethanol.

13. The method for producing a polyhydric alcohol according to any one of claims 1 to 12, the method comprising:
a step of hydroformylating dicyclopentadiene to obtain tricyclodecanedicarbaldehyde as the polyhydric aldehyde; and
a step of subjecting the tricyclodecanedicarbaldehyde to a reduction reaction in the presence of a hydrogenation catalyst and hydrogen to obtain a crude reaction solution containing tricyclo[5.2.1.0(2,6)]decane dimethanol.
